(19) Europäisches Patentamt
European Patent Office
Office européen des brevets

(11) EP 2 448 895 B1

(12) # FASCICULE DE BREVET EUROPEEN

(45) Date de publication et mention de la délivrance du brevet:
**01.05.2013 Bulletin 2013/18**

(21) Numéro de dépôt: **10734156.2**

(22) Date de dépôt: **10.06.2010**

(51) Int Cl.:
***C07C 29/20*** *(2006.01)* ***C07C 35/08*** *(2006.01)*
***C07C 35/02*** *(2006.01)*

(86) Numéro de dépôt international:
**PCT/FR2010/000425**

(87) Numéro de publication internationale:
**WO 2011/001041 (06.01.2011 Gazette 2011/01)**

(54) **PROCÉDÉ CONTINU D'HYDROGÉNATION DE COMPOSÉS AROMATIQUES**

KONTINUIERLICHES VERFAHREN ZUR HYDRIERUNG AROMATISCHER VERBINDUNGEN

CONTINUOUS METHOD FOR THE HYDROGENATION OF AROMATIC COMPOUNDS

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **03.07.2009 FR 0903266**

(43) Date de publication de la demande:
**09.05.2012 Bulletin 2012/19**

(73) Titulaire: **Aet Group**
**06830 Gilette (FR)**

(72) Inventeurs:
- **MARIE, Sabrina**
**F-06830 Revest-Les-Roches (FR)**
- **TRANI, Alexandre**
**F-06510 Carros (FR)**
- **PILIA, Raimondo**
**F-06800 Cagnes Sur Mer (FR)**

(74) Mandataire: **Schmidt, Martin Peter**
**IXAS Conseil**
**15, rue Emile Zola**
**69002 Lyon (FR)**

(56) Documents cités:
**WO-A-03/010119 WO-A1-02/100536**
**DE-A1- 2 132 547 US-A- 5 942 645**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

Domaine technique de l'invention

La présente invention concerne un procédé continu d'hydrogénation catalytique de composés aromatiques, et notamment de phénols substitués. Ce procédé est utilisable en chimie fine pour la préparation industrielle de dérivés du cyclohexane.

Etat de la technique

Les dérivés du cyclohexane contiennent un élément structural, l'anneau de cyclohexane, qui fait partie de nombreuses molécules utilisées, souvent en quantités relativement faibles, comme spécialités ou substances actives dans de nombreuses secteurs de l'industrie, telles que dans les industries pharmaceutiques, alimentaires, cosmétiques et photographiques. L'anneau cyclohexane est assez difficile à synthétiser par cyclisation de chaînes aliphatiques. Alternativement, il peut être obtenu par une cycloaddition de type Diels-Alder. Il peut également être obtenu par l'hydrogénation des dérivés du benzène. En effet, les dérivés du benzène sont assez facilement disponibles, y compris en quantités industrielles, certains dérivés du benzène faisant partie de fractions issues de la distillation du pétrole. Cependant, l'hydrogénation de dérivés du benzène conduit souvent à un mélange d'isomères. A titre d'exemple, il est bien connu (voir par exemple: Streitwieser/Heathcock, « Introduction to Organic Chemistry », McMillan 1976, page 623) que l'hydrogénation du 4-méthylphénol en présence de Ni et à une température de 150°C donne un mélange de cis-4-méthylcyclohexanol et de trans-4-méthylcyclohexanol.

On connaît de nombreux procédés pour hydrogéner le benzène et les dérivés du benzène. Il s'agit en général de procédés sous haute pression d'hydrogène et à température élevée, qui se déroulent en présence d'un catalyseur. La réaction est exothermique. Un grand nombre de travaux publiés concernent l'amélioration des catalyseurs.

Certains des procédés de l'état de la technique sont exécutés en mode « batch », c'est-à-dire dans un réacteur statique de type autoclave agité, dans lequel le composé de départ est mis en contact avec de l'hydrogène sous pression et à une température supérieure à la température ambiante pendant un certain temps suffisant pour que la réaction soit complète. L'hydrogène peut être appliqué de manière statique ou sous la forme d'un flux traversant le réacteur. A la fin de la réaction, les produits de réaction sont récupérés.

A titre d'exemple, la demande de brevet français FR 2 398 709 (ANIC S.p.a.) décrit un procédé d'hydrogénation de composés aromatiques en présence d'un catalyseur en lit fixe, réalisé dans un autoclave à une température comprise entre 100°C et 200°C, et à une pression comprise entre 1 et 150 bars. Le brevet US 2,857,432 (Allied Chemical Corporation) décrit un procédé d'hydrogénation de phénol en cyclohexanone en présence d'un catalyseur de palladium finement divisé dans un réacteur continu à une température comprise entre 100 - 200°C ; le document enseigne qu'à haute pression, des produits secondaires se forment.

La demande de brevet français FR 2 217 293 (Diamond Shamrock Corp.) décrit un procédé d'hydrogénation de composés benzéniques réalisé dans un autoclave, en présence d'un catalyseur sous la forme d'une pâte ou d'un gâteau de filtration, à une pression comprise entre 10 et 100 bar et à une température comprise entre 70°C et 200°C.

Le brevet US 6,489,520 (Haarmann & Reimer GmbH) décrit un procédé d'hydrogénation sélective de 2-t-butylphénol en cis-t-butylcyclohexanol en présence d'un catalyseur dispersé, à une température comprise entre 50°C et 200°C, et préférentiellement entre 90°C et 130°C, et à une pression comprise entre 1 et 100 bar, et préférentiellement entre 10 et 20 bar. Cette réaction est très longue, elle dure entre 2 à 100 heures.

La demande de brevet EP 0 703 210 A1 (Fuji Photo Film Co., Ltd.) décrit un procédé d'hydrogénation en présence d'un catalyseur dispersé, convenant à la préparation de nombreux cyclohexanols substitués à partir des composés phénoliques correspondants. Ce procédé se déroule en autoclave en présence de solvant ou sans solvant, à une température comprise entre 50°C et 300°C, préférentiellement entre 60°C et 250°C, et à une pression comprise entre 15 bar et 250 bar, préférentiellement entre 15 bar et 250 bar. La demande de brevet EP 0 427 965 (Firmenich & Cie) décrit un procédé continu d'hydrogénation du 4-t-butyl-phénol en 4-t-butyl-cyclohexanol en présence d'un système catalytique constitué de rhodium sur un support en combinaison avec du $HBF_4$ ou un de ses dérivés organiques, dans une proportion bien précise, à une température préférée de 40°C à 130°C, en présence d'un solvant, dans un réacteur de type autoclave à une pression comprise entre 3 et 200 bar.

On connaît également des procédés continus, dans lesquels un flux continu de composés de départ est traité avec de l'hydrogène, lesdits composés de départ entrent dans le réacteur, traversent le réacteur au cours de leur hydrogénation, et quittent le réacteur, le tout en flux continu.

Les brevets US 5,874,648 et US 6,031,140 (Bayer AG) décrivent un procédé d'hydrogénation d'un isocamphylguaiacol, qui est un compose comportant un anneau benzénique substitué par un groupement -OH (et possiblement un groupement $-OCH_3$ en position ortho par rapport au groupement -OH) et par un groupement isocamphyl, en isocamphylcyclohexanol. Cette réaction peut être effectuée en phase liquide, en une seule étape, avec ou sans solvant, à une

température comprise entre 140°C et 280°C, et préférentiellement entre 180°C et 250°C, à une pression d'hydrogène comprise entre 50 bar et 400 bar, et préférentiellement entre 150 bar et 300 bar, en présence d'un catalyseur en lit fixe.

Le brevet US 5,942,645 (BASF AG) décrit un procédé d'hydrogénation de composés comportant un anneau benzénique substitué par un groupement -OH et possiblement par un reste alkyl en C1 à C10, substitué ou non, et /ou par un radical alkoxy, en les composés cycloaliphatiques correspondants. Ce procédé se déroule en présence d'un catalyseur en lit fixe comportant au moins du ruthénium, et ce catalyseur est déposé sur un support poreux. La réaction peut être conduite sans solvant, mais la présence d'un solvant est préférée. La pression d'hydrogène est d'au moins 50 bar et de préférence comprise entre 150 bar et 300 bar. La température se situe entre 100°C et 270°C, et de préférence entre 150°C et 220°C. La réaction peut être conduite en mode continu.

Le brevet US 5,189,233 (Texaco Chemical Company) décrit un procédé d'hydrogénation de benzène dans lequel le benzène est exposé d'abord à un premier catalyseur en lit fixe d'activité modérée, puis le mélange réactionnel est exposé à un second catalyseur en lit fixe, plus actif que le premier. Le procédé est conduit à une température comprise entre 40°C et 300°C, de préférence entre 75°C et 230°C, et à une pression comprise entre 35 bar et 275 bar, sans solvant, dans un réacteur tubulaire à flux continu. Cette approche d'hydrogénation en deux étapes effectuée dans un réacteur continu, utilisant deux catalyseurs à lit fixe qui se distinguent par leur activité, se trouve également dans le brevet US 4,551,564 (Chemische Werke Hüls AG), qui cherche à améliorer le rendement en isomères cis de 2- et 4-t-butyl-cyclohexanol.

La demande de brevet français FR 2 040 257 (Badische Anilin- & Soda-Fabrik AG) décrit un procédé d'hydrogénation d'hydrocarbures aromatiques en cycloaliphatiques, avec ou sans solvant, à une température comprise entre 100°C et 400°C, et préférentiellement entre 150°C et 300°C, à une pression comprise entre 20 et 700 bar, et plus particulièrement entre 50 et 400 bar, dans un réacteur tubulaire, en mode continu et en présence d'un catalyseur en lit fixe.

Le brevet US 3,700,742 (Universal Oil Products Co.) décrit un procédé continu d'hydrogénation de fractions d'hydrocarbures aromatiques, telles qu'elles résultent de procédés de distillation du pétrole, en présence d'un catalyseur en lit fixe, à une pression comprise entre 35 bars et 140 bars et à une température comprise entre 90°C et 425°C. Ce document se situe dans un contexte qui est différent de celui de la chimie fine visant l'obtention de fractions pures de produits bien définis.

Les réacteurs de type batch et les réacteurs continus se distinguent fortement, pour des raisons techniques et pour des raisons pratiques. Sur le plan technique, pour une réaction exothermique telle que l'hydrogénation de composés aromatiques, le transfert de chaleur est un paramètre critique, à la fois pour la conception du réacteur et pour la conduite du procédé. C'est surtout pour un réacteur de type batch que l'augmentation de la taille du réacteur (« scaling-up »), et donc l'augmentation de sa capacité de production, est limitée par le transfert thermique. Plus on augmente la taille du réacteur pour gagner en productivité, plus le transfert thermique se dégrade. Dans des réacteurs de grande capacité, il est souvent nécessaire de diluer les réactifs par ajout de solvant pour être capable de contrôler l'exothermie de la réaction. Cependant, l'ajout d'un solvant nécessite ensuite une étape de séparation pour isoler le produit de la réaction du solvant.

Sur le plan pratique, un procédé discontinu nécessite à la fin de la réaction la dépressurisation du réacteur, son refroidissement, son ouverture, et sa vidange pour récupérer le mélange réactionnel dans des bonnes conditions de sécurité ; ces opérations discontinues sont complexes et coûteuses en main d'oeuvre. En plus, le coût d'investissement d'un réacteur de type autoclave est élevé. On aurait donc tendance à préférer un procédé continu, au moins pour des produits que l'on veut fabriquer en une quantité assez importante. Le transfert de masse est un des problèmes généraux des réacteurs chimiques, et des réacteurs d'hydrogénation en particulier. En mode discontinu (mode « batch »), des systèmes d'agitation ont été développés spécifiquement pour améliorer le transfert de masse. En mode continu, sans agitation, le transfert de masse peut être relativement peu efficace.

Dans les deux types de procédés, l'utilisation de solvants présente des inconvénients d'ordre énergétique (coût de la séparation, chauffage d'une masse plus grande), environnemental (rejets) et économiques (coût de l'investissement de l'étape de séparation, coût du solvant, diminution des vitesses de réaction).

La présente invention vise donc un procédé continu de préparation de dérivés du cyclohexane à partir de composés benzéniques, qui soit simple, facile à contrôler, à forts rendement et sélectivité, et qui évite l'ajout et la séparation ultérieure de solvant.

<u>Objets de l'invention</u>

L'invention a pour objet un procédé continu d'hydrogénation catalytique d'un composé aromatique, de préférence un dérivé mono- ou polysubstitué du benzène, en composé cyclo-aliphatique, ledit procédé étant exécuté dans un réacteur piston, de préférence de forme cylindrique, ledit réacteur étant pourvu d'un moyen mécanique d'agitation axiale, et dans lequel

- on fait entrer, de manière continue, de préférence à une extrémité dudit réacteur, au moins une phase liquide

comportant ledit composé aromatique et un catalyseur dispersé en phase liquide,

- on soumet ladite phase liquide, à une température comprise entre 100°C et 300°C et sous agitation mécanique axiale, à l'influence d'une pression d'hydrogène comprise entre 10 et 250 bar, et préférentiellement entre 50 et 250 bar, en présence dudit catalyseur dispersé en phase liquide, pendant un temps de passage t compris entre 10 secondes et 10 minutes, préférentiellement entre 10 secondes et 6 minutes, et plus préférentiellement entre 40 secondes et 3 minutes,
- on sort la phase liquide dudit réacteur,

et dans lequel, de manière préférée, l'augmentation de température $\Delta T$ du liquide entre l'entrée et la sortie du réacteur est telle que le rapport $\Delta T$ / $\Delta T_{ad}$ (où $\Delta T_{ad}$ représente l'augmentation adiabatique de température) est compris entre 0,02 et 0,6 lorsque le rapport entre le temps caractéristique de transfert de chaleur $t_{therm}$ et le temps caractéristique de transfert de matière $t_{mat}$ est compris entre 1 et 50. Ce procédé est utilisé de préférence sans solvant, i.e. ledit composé aromatique constitue la phase liquide qui entre dans le réacteur.

Description détaillée

L'invention concerne un procédé continu de réduction (ou hydrogénation) catalytique d'un composé aromatique, de préférence un dérivé mono- ou polysubstitué du benzène, en composé cyclo-aliphatique, ledit procédé étant exécuté dans un réacteur continu de type réacteur piston (appelé aussi réacteur à écoulement piston), de longueur L et de volume V, dans lequel les espèces chimiques (dérivé benzénique, catalyseur et hydrogène moléculaire) entrent à une extrémité et se déplacent tout au long du réacteur en se transformant progressivement. Le réacteur a de préférence une forme cylindrique. Il doit être pourvu d'un moyen d'agitation axiale, et de préférence d'un moyen mécanique d'agitation axiale. On entend ici par moyen d'agitation axiale tout dispositif qui assure une agitation du mélange réactionnel sur toute la longueur, ou partie significative de celle-ci, par un moyen possédant un axe parallèle à l'axe du réacteur. Ce moyen d'agitation axiale facilite, d'une part, le déroulement de la réaction, en mélangeant les espèces chimiques entrant avec le catalyseur, qui se trouve sous forme dispersée dans une phase liquide, et facilite d'autre part le transfert thermique.

Le réacteur piston possède un profil de température et de concentration qui peuvent varier le long de son axe. Un tel réacteur peut être modélisé comme une suite de réacteurs élémentaires disposés en série le long d'un axe et ayant chacun une longueur $\Delta L$ et un volume $\Delta V$. Dans les conditions de fonctionnement de ce réacteur, la composition de l'alimentation et le débit volumique total F sont uniformes et constants, et le temps de séjour

$$\tau = V/F \qquad \text{(Equation 1)}$$

est constant pour toutes les molécules entrant dans le réacteur. Ce type de réacteur est connu, et l'homme du métier sait également que si l'on réalise une réaction très exothermique dans un réacteur piston, le transfert radial de chaleur peut devenir limitant. Pour l'hydrogénation des dérivés benzéniques, qui implique la saturation d'au moins trois liaisons doubles conjuguées, la maîtrise des transferts thermiques est donc critique.

Le procédé selon l'invention implique une réaction chimique de type

$$\text{A (liquide)} + \nu \text{ B (gaz)} \rightarrow \nu_p \text{ Produit} \qquad \text{(Equation 2)}$$

où v est le coefficient stoechiométrique du gaz et $\nu_p$ est le coefficient stoechiométrique du produit. Selon l'invention, le gaz B est de l'hydrogène, et le dérivé benzénique à hydrogéner se présente sous la forme d'un liquide pur ou dilué dans un solvant liquide, ou sous la forme d'un solide dilué dans un solvant liquide.

D'une façon générale, les performances des réacteurs sont données par deux grandeurs caractéristiques, qui décrivent respectivement le transfert thermique et le transfert de matière. Ces temps caractéristiques de transfert sont définies ci-dessous par des équations simplifiées (le modèle hydrodynamique étant le même, que le réacteur soit un réacteur piston ou un réacteur parfaitement agité, dans la mesure où la réaction d'hydrogénation est limitée par le transfert de matière) :

- le temps caractéristique de transfert de chaleur

$$t_{therm} = \frac{\rho C_p V_{liq}}{K S} \qquad \text{(Equation 3)}$$

- le temps caractéristique de transfert de matière

$$t_{mat} = 1/(k_L a) \qquad \text{(Equation 4).}$$

Dans ces équations, les paramètres suivants sont utilisés :

- la masse volumique du liquide $\rho$;
- la capacité calorifique du liquide $C_p$ ;
- le coefficient global de transfert K, défini ci-dessous ;
- la surface d'échange thermique S (constante pour un réacteur donnée, car fixée par sa conception) ;
- le produit entre le coefficient de transfert de matière gaz-liquide côté liquide, $k_L$, et l'aire interfaciale spécifique, a, définie ci-dessous.

Plus le temps caractéristique de transfert est petit, plus le système est performant et transfère rapidement la chaleur et la matière (respectivement).
Nous décrivons ici de manière sommaire la détermination du coefficient K bien connue de l'homme du métier.
Le coefficient global de transfert K (appelé aussi coefficient d'échange global) est défini par l'équation

$$\Phi = K\, S\, \Delta T_{ml} \qquad \text{(Equation 5)}$$

où S est la surface d'échange (en l'espèce S = $\pi$ D L où D est le diamètre intérieur et L la longueur intérieure de la partie du tube du réacteur dans laquelle le gaz entre en contact avec le liquide), $\Delta T_{ml}$ est la différence de température moyenne logarithmique :

$$\Delta T_{ml} = \{[(T(\text{caloporteur})_{sortie} - T(\text{procédé})_{entrée}] - [(T(\text{caloporteur})_{entrée} - T(\text{procédé})_{sortie}]\} / \ln\{([(T(\text{caloporteur})_{sortie} - T(\text{procédé})_{entrée}] / [(T(\text{caloporteur})_{entrée} - T(\text{procédé})_{sortie}]\}$$

et $\Phi$ est la puissance (en Watts, température de référence 25°C) gagnée par le flux de chaleur côté procédé. Pour une réaction donnée, ces paramètres dépendent de la géométrie du réacteur et du débit ; ils peuvent être déterminés aisément.
Le coefficient $k_L a$, également bien connu de l'homme du métier, peut être déterminé expérimentalement par une procédure qui, afin de ne pas alourdir inutilement la description de l'invention, est décrite ci-dessous comme « Exemple 1 ».
Dans un mode de réalisation avantageux de l'invention, on utilise un réacteur continu de type piston possédant les caractéristiques suivantes :

- Transfert de matière : $0{,}1\ s^{-1} < k_{LA} < 0{,}3\ s^{-1}$ soit $3\ s < t_{mat} < 10\ s$
- Transfert de chaleur: K = 300 à 1000 W /$m^2$ /°C (préféré : 700 W /$m^2$ /°C, et encore plus préférentiellement : environ 550 W /$m^2$ /°C)

(on considère ici le coefficient de transfert partiel du liquide avec le métal).

Dans un mode de réalisation typique, le temps caractéristique de transfert $t_{therm} = \frac{\rho C_p V_{liq}}{K S}$ est de l'ordre de 25 secondes (avec p=1050 kg/$m^3$ Cp=2000 J/kg/°C).
Dans ce mode de réalisation avantageux, le rapport des temps caractéristiques est donc : $2 < (t_{therm}/t_{mat}) < 8$

Dans le procédé selon l'invention, l'augmentation de température du liquide $\Delta T$ entre l'entrée et la sortie du réacteur est telle que :

$$\boxed{\frac{\Delta T}{\Delta T_{ad}} = \frac{t_{therm}}{(t_{therm} + \tau_{liq})} X_A} \qquad \text{(Equation 6)}$$

où

- $\Delta T_{ad}$ est l'augmentation adiabatique de température

$$\Delta T_{ad} = \frac{(-\Delta_r H) C_{A0}}{\rho C_p} \qquad \text{(Equation 7)}$$

- $\Delta_r H$ est l'enthalpie de la réaction,
- $X_A$ est le coefficient stoechiométrique du composé A.

Pour le cas d'une conversion totale de A (i.e. $X_A = 1$) on peut réécrire l'équation (6) en

$$\boxed{\frac{\Delta T}{(\Delta T_{ad})} = \frac{M}{(t_{mat} / t_{therm} + 1)}} \qquad \text{(Equation 8)}$$

où M désigne le rapport stoechiométrique :

$$M = \frac{P}{\nu He C_{A0}} \qquad \text{(Equation 9)}$$

dans lequel

- P signifie la pression de travail
- He signifie coefficient de Henry
- $C_{A0}$ signifie la concentration du liquide à l'entrée du réacteur.

Le choix des conditions opératoires du procédé selon l'invention fait intervenir trois grandeurs :

- l'augmentation adiabatique de température en milieu non dilué

$$(\Delta T_{ad})_{pur} = \frac{(-\Delta_r H)(C_{A0})_{pur}}{\rho C_p} \qquad \text{(Equation 10)}$$

- le rapport stoechiométrique calculé sur la concentration des réactifs purs

$$M_{pur} = \frac{P}{\nu He (C_{A0})_{pur}} \qquad \text{(Equation 11)}$$

- le facteur de dilution F défini par

$$(C_{A0})_{travail} = \frac{(C_{A0})_{pur}}{F} \qquad \text{(Equation 12).}$$

Les inventeurs ont découvert qu'un régime de fonctionnement particulier d'un réacteur piston permet de résoudre le problème posé. Ce régime est expliqué ici dans le cas d'une réaction avec un coefficient stoechiométrique $\nu p = 1$, comme c'est le cas par exemple pour l'hydrogénation de l'ortho-crésol.

Le procédé continu peut être décrit comme comportant plusieurs étapes. Dans une première étape, on fait entrer, de manière continue, de préférence à une extrémité dudit réacteur, une phase liquide comportant ledit composé aromatique et le catalyseur dispersé. Puis on soumet ladite phase liquide (suspension) à une température comprise entre 100°C et 300°C et sous agitation mécanique axiale, à l'influence d'une pression d'hydrogène comprise entre 10 et 250 bar (préféré : entre 50 et 250 bar) pendant un temps de passage t compris entre 1 seconde et 10 minutes (préféré : 10 secondes et 6 minutes, et encore plus préférentiellement 40 secondes à 3 mnutes). Lorsque la phase liquide est arrivée à l'autre extrémité du réacteur, on la sort à l'autre extrémité du réacteur, puis on sépare le catalyseur par filtration.

De manière préférée, on conduit la réaction de manière à ce que l'augmentation de température $\Delta T$ du liquide entre l'entrée et la sortie du réacteur soit telle que le rapport $\Delta T / \Delta T_{ad}$ (où $\Delta T_{ad}$ représente l'augmentation adiabatique de température) est compris entre 0,02 et 0,6 lorsque le rapport entre le temps caractéristique de transfert de chaleur $t_{therm}$ et le temps caractéristique de transfert de matière $t_{mat}$ est compris entre 1,5 et 50. Ce procédé est utilisé de préférence sans solvant, i.e. ledit composé aromatique constitue la phase liquide qui entre dans le réacteur. Dans un mode de réalisation préféré, le rapport $\Delta T / \Delta T_{ad}$ est compris entre 0,02 et 0,2 lorsque $t_{therm} / t_{mat}$ est compris entre 1,5 et 12. Dans un mode de réalisation encore plus préféré, $\Delta T / \Delta T_{ad}$ est compris entre 0,03 et 0,15 lorsque $t_{therm} / t_{mat}$ est compris entre 2 et 8.

Pour maintenir constante la température interne du réacteur, on ajuste la puissance de chauffage de la chambre de réaction, sachant qu'une partie de l'énergie thermique nécessaire pour la maintenir à la température sélectionnée provient de l'enthalpie de la réaction d'hydrogénation qui se déroule dans la chambre de réaction.

Avantageusement, le procédé selon l'invention est mis en oeuvre dans un réacteur piston tubulaire de forme cylindrique d'un diamètre intérieur compris entre 20 mm et 100 mm. Au-dessus de 100 mm, la productivité du réacteur diminue car pour que la surface d'échange reste importante, il faut diminuer le débit. Au-dessous de 20 mm, le rapport surface / volume est très important, mais le débit est insuffisant pour une production industrielle. De manière préférée, le diamètre intérieur du réacteur piston est comprise entre 30 mm et 75 mm, et de manière encore plus préférée entre 40 mm et 60 mm. La longueur de la chambre de réaction du réacteur est comprise entre 10 cm et 100 cm. Au-dessous de 10 cm, le temps de séjour est trop court. Au-dessus de 100 cm, l'usinage du réacteur tubulaire devient difficile, et l'agitation du mélange réactionnel est difficile à accomplir. Une longueur préférée se situe entre 20 cm et 80 cm.

Le réacteur doit être pourvu d'un moyen mécanique d'agitation axiale. Différents moyen sont utilisables à cette fin, tels qu'une suite de malaxeurs, une vis sans fin, une hélice, mais ce moyen mécanique d'agitation axiale ne doit pas perturber le caractère « piston » du réacteur, tel que défini par l'équation (1).

Le procédé selon l'invention met en oeuvre au moins un catalyseur dispersé, tel qu'une poudre en suspension. Avantageusement, cette poudre est constituée d'un support (tel que l'alumine, la silice ou le charbon actif) sur lequel a été préalablement déposé un élément métallique. A titre d'exemple, on peut utiliser le Pd, Ru, Pt, Ni sur un support de charbon actif.

Par rapport aux procédés en mode batch, le procédé selon l'invention a de nombreux avantages. Souvent, la quantité de catalyseur nécessaire peut être réduite de manière significative, souvent d'un facteur 5 à 20. Pour de nombreuses réactions, on peut trouver des conditions opératoires dans lesquelles le rendement de la réaction est égal ou supérieur à 99%. Ce rendement élevé permet d'éviter des étapes de purification supplémentaires qui sont souvent nécessaires en chimie fine lorsque le rendement est inférieur à 95%. On peut travailler dans de nombreux cas sans solvant. Le réacteur permet de produire des quantités industrielles de dérivées de cyclohexane, par exemple de l'ordre de 20 kg/h dans le cas de l'ortho-crésol. Cela permet d'accéder à une production annuelle de l'ordre de 80 à 100 tonnes avec un seul réacteur. Par ailleurs, le coût d'investissement d'un réacteur capable de mettre en oeuvre le procédé selon l'invention est plus faible que celui pour un réacteur de type batch, et le besoin en main d'oeuvre est réduit. Par ailleurs, le « scaling up » du procédé est fortement simplifié puisque le procédé selon l'invention peut être mis en oeuvre dans un réacteur continu industriel de petite taille, qui ne diffère pas beaucoup d'un réacteur expérimental de laboratoire. Et finalement, le procédé n'utilise en général pas de solvant et ne produit en général pas de déchet.

Le procédé selon l'invention est applicable à de nombreux composés chimiques. D'une manière générale, il permet l'hydrogénation d'un composé aromatique, de préférence un dérivé mono- ou polysubstitué du benzène, en

composé cycloaliphatique. Plus particulièrement, le procédé est applicable aux dérives mono- ou polysubstitués du phénol de la formule (I), et il conduit ainsi aux molécules de la formule (II). Des exemples pour les molécules de la formule (I) sont les crésols (ortho-, méta- et para-crésol) et le guaiacol.

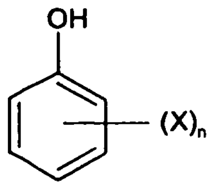
(I)

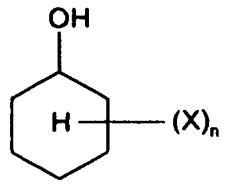
(II)

On peut aussi hydrogéner des anneaux benzéniques portant plusieurs groupe hydroxyle, tels que les dihydroxybenzènes (notamment : catéchol, résorcinol, hydroquinone) et les trihydroxybenzènes (notamment le pyrogallol, le phloroglucinol et l'acide gallique).

D'autres exemples pour des anneaux benzéniques à hydrogéner sont les acides hydroxybenzoiques (tels que l'acide salicylique) et les nitrophénols (tels que l'acide picrique).

Le procédé selon l'invention est aussi applicable aux molécules de la formule (III). Dans ce cas, il conduit aux molécules de la formule (IV).

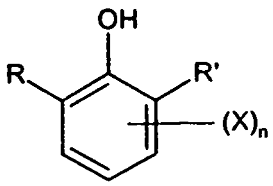
(III)

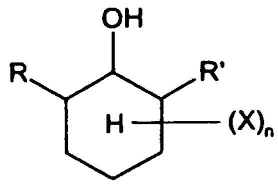
(IV)

Dans les formules (I), (II), (III) et (IV), X représente un substituant qui n'est pas un atome hydrogène. X peut être par exemple un groupe aliphatique, linéaire ou ramifié, ou un groupe aralkyle, un groupe alkenyle, un groupe cycloalkenyle, un groupe cycloalkyle, un groupe cycloalkenyle, tous ces groupes comprenant avantageusement entre 1 à 36 atomes de carbone. Comme exemples spécifiques, on indique ici les groupes méthyle, éthyle, propyle, isopropyle, t-butyle, tridécyle, cyclohexyle, 4-pentadécyloxybenzyle, hexadécyloxycarbonyléthyle, 2-éthoxytridécyle, trifluoro-méthyle et cyclopentyle.

X peut aussi être un groupe alkoxy (de préférence avec 1 à 30 atomes de carbone, tel que méthoxy, éthoxy, 2-méthylméthoxy et 2-dodécyloxyéthoxy), un groupe alkyle, un groupe carbamoyloxy (de préférence avec 1 à 30 atomes de carbone, tel que N-ethyl-carbamoyloxy et N-phénylcarbamoyloxy), un groupe silyloxy (de préférence avec 1 à 30 atomes de carbone, tel que triméthylsilyloxy et dibutylméthylsilyloxy), un groupe acylamino (de préférence avec 2 à 30 atomes de carbone tel que acétamide, tetradécanamide, 2-(2,4-di-t-amylphénoxy)-acétamide, isopentadécanamide), un groupe alkylamino (de préférence avec 1 à 30 atomes de carbone, tel que méthylamino, butylamino, dodécylamino, diméthylamino, diéthylamino et méthylbutylamino), un groupe uréide (de préférence avec 2 à 30 atomes de carbone, tel que méthyluréide, phényluréide, N,N-dibutyluréide et diméthyluréide), un groupe alkényloxy (de préférence avec 2 à 30 atomes de carbone, tel que 2-propenyloxy), un groupe formyle, un groupe alkylacyle, un groupe alkyl oxycarbonyl, un groupe alkyl oxycarbonylamino ou un groupe carbamoyle (de préférence avec 1 à 30 atomes de carbone tel que N-éthylcarbamoyle, N,N-dibutylcarbamoyle, N-(2-dodécyloxyéthyl)carbamoyle, N-méthyl-N-dodécylcarbamoyl et N-[3-(2,4-di-t-amylphénoxy)propyl]carbamoyle), un groupe phosphonyle (de préférence avec 1 à 30 atomes de carbone tel que phénoxyphosphonyle, octyloxy-phosphonyle et phénylphosphonyle), un groupe imide (de préférence avec 1 à 30 atomes de carbone tel que N-succinimide, hydantoinyl, N-phthalimide et 3-octadécenyl-succin-imide), un groupe azolyle (tel qu'imidazolyle, pyrazolyle, 3-chloro-pyrazol-1-yl et triazolyle), un atome halogène (tel que chlore et brome), un groupe hydroxyle, un groupe cyanure, un groupe carboxyle, un groupe nitro, un groupe amine linéaire, et des groupes analogues.

De manière préférée, X est un groupe alkyle linéaire ou ramifié avec 1 à 15 atomes de carbone et encore plus préférentiellement avec 1 à 8 atomes de carbone, un groupe aralkyle avec 7 à 15 atomes de carbone et encore plus préférentiellement avec 7 ou 8 atomes de carbone, ou un groupe cycloalkyle avec 3 à 15 atomes de carbone et encore plus préférentiellement avec 5 à 8 atomes de carbone. De manière particulièrement préférée, X est un groupe méthyle, éthyle, n-propyle, i-propyle, n-butyle, s-butyle, t-amyle, t-octyle, phényle ou cyclohexyle. Le groupe méthyle est le plus préféré de tous. Le paramètre n, un nombre entier, est de préférence 0 ou 1, et lorsque n est 1, X est de préférence situé en position para par rapport au groupe hydroxyle dans les formules (III) et (IV).

Dans les formules (III) et (IV), R et R' sont indépendamment l'un de l'autre un groupe alkyle tertiaire. De manière plus spécifique, R et R' sont représentés par la formule (V), dans laquelle R1, R2 et R3 sont, indépendamment les uns

des autres, un substituant qui remplace le radical d'hydrogène. Ces substituants sont ceux indiqués pour le radical X, dans la mesure où ils peuvent être connectés à un atome de carbone.

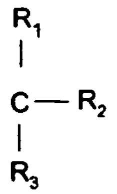

(V)

De manière préférée, R1, R2 et R3 sont chacun un groupe alkyle linéaire ou ramifié avec 1 à 15 atomes de carbone et encore plus préférentiellement avec 1 à 8 atomes de carbone, un groupe aralkyle avec 7 à 15 atomes de carbone et encore plus préférentiellement avec 7 ou 8 atomes de carbone, ou un groupe cycloalkyle avec 3 à 15 atomes de carbone et encore plus préférentiellement avec 5 à 8 atomes de carbone. Les groupes alkyle linéaires ou ramifiés avec 1 à 4 atomes de carbone sont préférés pour R1, R2 et R3, et le groupe méthyle est le plus préféré de tous. R1, R2 et R3 peuvent être identiques ou différents, et peuvent être reliés entre eux pour former un anneau.
Des exemples préférés pour R et R' comprennent les groupes t-butyle, t-amyle, t-octyle, 1-méthylcyclohexyle, et le groupe t-butyle est le plus préféré pour R et R'.

Les groupes R et R' se trouvant dans les deux positions ortho par rapport au groupe hydroxyle des molécules de la formule (III) et (IV) peuvent être identiques ou différents, mais il est préféré qu'ils soient identiques.
Lorsque R ou R' comportent un substituant susceptible d'être modifié lors de l'hydrogénation catalytique, par exemple s'il contient une liaison double ou un système aromatique, ce substituant dans la formule (IV) peut différer de celui dans la formule (III).
Dans les formules (III) et (IV), le paramètre n est un nombre entier compris entre 0 et 3. Lorsque n est 2 ou 3, X peut être le même ou différent, et plusieurs X peuvent être connectés pour former un anneau. Comme dans le cas de R et R' décrit ci-dessus, lorsque X comporte un substituant susceptible d'être modifié lors de l'hydrogénation catalytique, par exemple s'il contient une liaison double ou un système aromatique, ce substituant dans la formule (IV) peut différer de celui dans la formule (III). Par ailleurs, lorsque X devient, suite à l'hydrogénation catalytique, un atome d'hydrogène, le paramètre n peut devenir un nombre différent. De manière préférée, n est égal à 1. Selon l'invention, des composes préférés représentés par les formules (III) et (IV) sont ceux dans lesquels R1, R2 et R3 dans la formule (V) sont indépendamment les uns de autres un groupe alkyle avec 1 à 15 atomes de carbone, un groupe aralkyle avec 7 à 15 atomes de carbone, un groupe cycloalkyle avec 3 à 15 atomes de carbone, ou un groupe aryle avec 6 à 15 atomes de carbone ; n est 1, et X est en position para par rapport au groupe hydroxyle dans les formules (III) et (IV).

Des composés plus préférés des formules (III) ou (IV) sont ceux dans lesquels à la fois R et R' sont des groupes t-butyle, t-amyle, t-octyle ou 1-méthylcyclohexyle ; X est un groupe méthyle, éthyle, n-propyle, iso-propyle, n-butyle, sec-butyle, t-amyle, t-octyle, phenyle ou cyclohexyle ; n est 1 ; et X se situe en position para par rapport au groupe hydroxyle dans les formules (III) et (IV). En particulier, les composes dans lesquels X est n groupe méthyle sont preférés. D'une manière plus générale, X représente ici un groupe aliphatique ou aryl, un groupe hétérocyclique, un groupe alkoxy, ou aryloxy, un groupe oxy hétérocyclique, un groupe alkylacyloxy ou arylacyloxy, un groupe acyloxy hétérocyclique, un groupe carbamoyloxy, silyloxy, acylamino, alkylamino, arylamino, ureido, alkenyloxy, formyl, alkylacyl ou arylacyl, un groupe, acyl hétérocyclique, un groupe alkyloxycarbonyl ou aryloxycarbonyl, un groupe oxycarbonyl hétérocyclique, un groupe alkyloxycarbonyl-amino ou aryoxycarbonylamino, un groupe oxycarbonylamino hétérocyclique, un groupe carbamoyl, phosphonyl, imide ou azolyl, un atome halogène, un groupe hydroxyl, cyano, carboxy ou nitro, ou un groupe amino nonsubstitué.

Exemples

L'invention est illustrée ci-dessus par les exemples 2 à 3 qui cependant ne limitent pas l'invention. L'exemple 1 concerne une réaction chimique qui sert ici pour décrire une procédure expérimentale utilisable pour déterminer le paramètre $k_La$ d'un réacteur piston. L'exemple 1 ne fait pas partie de l'invention.

Exemple 1 :

Nous indiquons ici une méthode expérimentale utilisable pour déterminer le paramètre $k_La$ d'un réacteur.

On détermine le produit $k_La$ pour un réacteur donné à partir d'une réaction chimique parfaitement connue, à savoir l'hydrogénation catalytique du nitrobenzène en aniline ($Ph\text{-}NO_2 + 3\ H2 \rightarrow Ph\text{-}NH_2 + 2\ H_2O$, où Ph désigne un groupe phényle). On effectue cette réaction en phase liquide sans solvant, la phase gazeuse étant constituée d'hydrogène

pur à une pression initiale de 2 bars. Le catalyseur est constitué de carbone pulvérulent (diamètre de particule équivalent de l'ordre de 50 $\mu$m) chargé à 5% en masse de palladium. La concentration massique du catalyseur est de 2,5 g/l et l'hydrogénation est effectuée à température ambiante. Un capteur de pression à quartz permet de mesurer la pression d'hydrogène en fonction du temps. Le réacteur dispose d'une double enveloppe ; une circulation d'eau thermostatée à l'intérieur de la double enveloppe permet de maintenir constante la température du réacteur. Au départ, le réacteur non agité est maintenu sous pression d'azote ; il est ensuite purgé à l'hydrogène. A une pression d'hydrogène de 2 bars, on met en route l'agitation et enregistre la baisse de la pression d'hydrogène. On laisse la réaction se poursuivre jusqu'à ce que la pression atteigne la valeur de 0,5 atm. Ensuite, on arrête l'agitation et repressurise l'appareil avec de l'hydrogène, puis on attend une dizaine de minutes et répète la mesure avec une vitesse d'agitation différente. Pour chaque essai effectué, on constate que la pression d'hydrogène décroit suivant une loi exponentielle. Ainsi, en traçant ln $P_{H2}/P_0$ = f (t), on obtient une droite dont la pente permet d'accéder au produit $\alpha$ $k_{app}$. Si l'on trace l'évolution de ce produit $\alpha$ $k_{app}$ avec la vitesse d'agitation on observe un comportement asymptotique. Pour de faibles vitesses d'agitation, la conductance apparente augmente avec la vitesse d'agitation ; cela indique une limitation de la cinétique apparente pour le transfert gaz-liquide. Pour des fortes vitesses d'agitation, on atteint un plateau ; cela indique que le transfert est limité, soit par la cinétique chimique soit par la cinétique du transfert de matière liquide - solide. L'exploitation de la courbe $\alpha$ $k_{app}$ = f(vitesse d'agitation) permet alors d'estimer la valeur de la conductance de transfert $k_L$ a. En pratique, cinq à dix points expérimentaux sont suffisants pour déterminer cette valeur.

Nous indiquons ici les bases théoriques de cette détermination de $k_L$a.

En négligeant l'accumulation de l'hydrogène en phase liquide, on peut établir l'expression du flux de disparition de l'hydrogène en réacteur fermé.

$$\frac{dn_{H_2}}{dt} = -\frac{V_G}{RT}\frac{dP_{H_2}}{dt} = \varphi_{H_2} V_R \qquad (1)$$

où $\varphi_{H2}$ est le flux spécifique de disparition de l'hydrogène. Ce flux peut être exprimé en faisant apparaître ou bien la vitesse de réaction ou bien le flux de transfert :

$$\varphi_{H_2} = r_V \alpha = \left(K_{H_2} a\right)_{global}\left(\frac{P_{H_2}}{He} - C_{H_2}^{surface}\right) \qquad (2)$$

$r_V$ est la vitesse de réaction volumique de l'hydrogénation, $\alpha$ la rétention de solide dans le réacteur et $(K_{H2}a)_{global}$ la conductance globale de transfert de l'hydrogène de la phase gazeuse vers la surface du catalyseur.

Le volume du catalyseur et la concentration en nitrobenzène étant considérés comme invariants au cours d'un essai, la vitesse de la réaction peut être exprimée comme résultant d'une cinétique de premier ordre par rapport à la concentration en hydrogène, soit :

$$r_V = \eta k_V C_{NB} C_{H_2}^{surface} = k_V' C_{H_2}^{surface} \qquad (3)$$

Lorsque la phase gazeuse est de l'hydrogène pure, la conductance globale de transfert peut être exprimée en fonction des conductances de transfert partielles gaz-liquide et liquide-solide par :

$$\frac{1}{\left[K_{H_2} a\right]_{global}} = \frac{1}{k_L a_{LG}} + \frac{1}{k_S a_S \alpha} \qquad (4)$$

où $a_s$ est la surface spécifique du solide et $a_{LG}$ la surface spécifique gaz-liquide. En combinant les expressions de la cinétique chimique et de la cinétique physique, le flux spécifique de disparition de l'hydrogène dans le réacteur peut

être exprimé par :

$$\varphi_{H_2} = \alpha k_{app} \frac{P_{H_2}}{He} \qquad (5)$$

où $\alpha$ $k_{app}$ est une conductance apparente qui intègre les limitations dues à la cinétique chimique, mais aussi les limitations dues à la cinétique physique.

$$\frac{1}{\alpha k_{app}} = \frac{1}{\left[K_{H_2}a\right]_{global}} + \frac{1}{\alpha k_v'} = \frac{1}{k_L a_{LG}} + \frac{1}{\alpha}\left[\frac{1}{k_S a_S} + \frac{1}{k_v'}\right] \qquad (6)$$

En injectant (5) dans (1) on obtient :

$$\frac{dP_{H_2}}{P_{H_2}} = -\alpha k_{app} \frac{V_R}{V_G} \frac{RT}{He} dt \qquad (7)$$

dont l'intégration conduit à :

$$\ln \frac{P_{H_2}}{P_0} = -\alpha k_{app} \frac{V_R}{V_G} \frac{RT}{He} (t - t_0) \qquad (8)$$

L'interprétation de l'évolution de la pression d'hydrogène dans un système fermé permet ainsi de déterminer la conductance apparente du système. Cette dernière permet de remonter à la valeur de la conductance de transfert gaz-liquide.

Exemple 2 :

L'utilisation du procédé selon l'invention pour hydrogénation catalytique de l'ortho-crésol a déjà été décrite ci-dessus en détail. Cette réaction a été effectuée sans solvant. Une suspension composée d'ortho-crésol fondu et de 0,4% massiques de catalyseur de type nickel sur support charbon est introduite dans le réacteur piston à l'aide d'une pompe. Le mélange est préchauffé en continu à l'extérieur du réacteur à une température d'environ 180°C. L'hydrogène est maintenu à une pression d'environ 150 bar. Pour un temps de séjour d'environ 2 minutes et trente secondes, la conversion dans ces conditions est supérieure à 99,9% En sortie du réacteur (température de sortie environ 280°C), après dépressurisation et filtration du catalyseur, le méthylcyclohexanol est obtenu avec une pureté supérieure à 99,0 % telle que vérifiée par chromatographie en phase gazeuse.

Exemple 3 :

On compare ici, pour le cas spécifique de l'hydrogénation de l'ortho-crésol, la productivité du procédé selon l'invention avec un procédé continu selon l'état de la technique.

Pour le réacteur batch, on utilise une cuve agitée d'hydrogénation de 6 $m^3$, remplie au 2/3 environ (soit environ 4000 litres). On utilise un mélange réactionnel constitué d'un volume d'ortho-crésol et 5 volumes d'éthanol en tant que solvant, soit environ 670 kg d'ortho-crésol pour environ 3350 litres d'éthanol. La réaction d'hydrogénation est conduite à 100°C sous une pression d'hydrogène inférieure à 10 bars pendant 4 heures, en présence d'un catalyseur, jusqu'à

conversion complète. Le temps du cycle complet est d'environ 30 heures, comprenant le chargement du réacteur, l'inertage (mise sous vide, rinçage à l'azote), la mise sous hydrogène (p < 10 bar), la réaction d'hydrogénation elle-même, le refroidissement, la filtration du catalyseur et la distillation du solvant. Pour 670 kg de ortho-crésol ajouté, on obtient 672 kg de méthylcyclohexanol et 34 kg d'ortho-crésol non-réagi. Cela correspond à un rendement de 95 % et à une productivité de 22,4 kg/h.

Avec le procédé selon l'invention, conduit sans solvant et en présence d'un catalyseur dispersé, dans un réacteur continu dont le volume de liquide réactionnel est $V_{liq}$ =0,7 litres, on obtient une productivité (à 150 bars avec un temps de transfert de matière $t_{mat}$ =3,5 s) de l'ordre de 5,5 x $10^{-2}$ mol/s, soit un débit massique de cyclohexanol (M=114 g/mol) de l'ordre de 22 kg/h.

Pour un réacteur continu, la productivité du procédé selon l'invention telle que déterminée ci-dessus est tout à fait intéressante sur le plan industriel, même dans le cas d'une molécule simple comme l'ortho-crésol ; elle l'est encore plus pour des molécules plus compliquées. Pour accroître la productivité, on peut augmenter le diamètre du réacteur, mais cette possibilité est limitée par le transfert de chaleur, comme expliqué ci-dessus. Avantageusement, on utilise une pluralité de réacteurs, compte tenu de leur simplicité, du caractère continu du procédé et du fait que ce procédé continu ne nécessite pas l'intervention de beaucoup de main d'oeuvre.

## Revendications

1. Procédé continu d'hydrogénation catalytique d'un composé aromatique, de préférence un dérivé mono- ou polysubstitué du benzène, en composé cyclo-aliphatique, ledit procédé étant exécuté dans un réacteur piston, de préférence de forme cylindrique, ledit réacteur étant pourvu d'un moyen mécanique d'agitation axiale,
et dans lequel

    - on fait entrer, de manière continue, de préférence à une extrémité dudit réacteur, au moins une phase liquide comportant ledit composé aromatique et un catalyseur dispersé en phase liquide,
    - on soumet ladite phase liquide, à une température comprise entre 100°C et 300°C et sous agitation mécanique axiale, à l'influence d'une pression d'hydrogène comprise entre 10 et 250 bar, et préférentiellement entre 50 et 250 bar, en présence dudit catalyseur dispersé en phase liquide, pendant un temps de passage t compris entre 1 seconde et 10 minutes, préférentiellement entre 10 secondes et 6 minutes, et plus préférentiellement entre 40 secondes et 3 minutes,
    - on sort la phase liquide dudit réacteur, ,

    et dans lequel, de manière préférée, l'augmentation de température $\Delta T$ du liquide entre l'entrée et la sortie du réacteur est telle que le rapport $\Delta T$ / $\Delta T_{ad}$ (où $\Delta T_{ad}$ représente l'augmentation adiabatique de température) est compris entre 0,02 et 0,6 lorsque le rapport entre le temps caractéristique de transfert de chaleur $t_{therm}$ et le temps caractéristique de transfert de matière $t_{mat}$ est compris entre 1 et 50.

2. Procédé selon la revendication 1, **caractérisé en ce que** $\Delta T$ / $\Delta T_{ad}$ est compris entre 0,02 et 0,2 lorsque $t_{therm}$ / $t_{mat}$ est compris entre 1,5 et 12.

3. Procédé selon la revendication 1, **caractérise en ce que** $\Delta T$ / $\Delta T_{ad}$ est compris entre 0,03 et 0,15 lorsque $t_{therm}$ / $t_{mat}$ est compris entre 2 et 8.

4. Procédé selon l'une quelconque des revendications 1 à 3, dans lequel 3 s < $t_{mat}$ < 10 s.

5. Procédé selon l'une quelconque des revendications 1 à 4, dans lequel le transfert de chaleur est compris entre 300 et 700 W/$m^2$/°C.

6. Procédé selon une quelconque des revendications 1 à 5, dans lequel ledit composé aromatique constitue la phase liquide qui entre dans le réacteur.

7. Procédé selon une quelconque des revendications 1 à 5, dans lequel ledit composé aromatique est un phénol mono- ou polysubstitué, ou un polyhydroxybenzène non substitué ou un polyhydroxybenzène mono- ou polysubstitué.

8. Procédé selon la revendication 7, dans lequel ledit composé aromatique est tel que représenté par la formule (I), et ledit composé cyclo-aliphatique est tel que représenté par la formule (II),

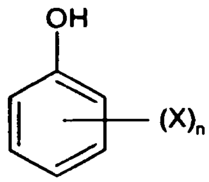 (I) 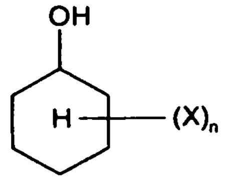 (II)

où X est un substituant qui n'est pas un atome d'hydrogène, et n est un nombre entier compris entre 0 et 3.

**9.** Procédé selon la revendication 7, dans lequel ledit composé aromatique est tel que représenté par la formule (III), et ledit composé cyclo-aliphatique est tel que représenté par la formule (IV),

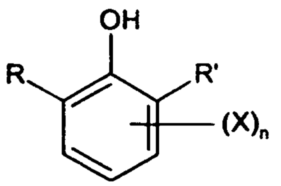 (III) 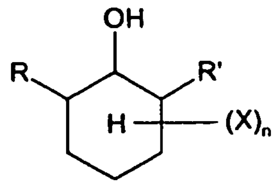 (IV)

où R et R' sont, indépendamment l'un de l'autre, un groupe alkyle tertiaire, X est un substituant qui n'est pas un atome d'hydrogène, et n est un nombre entier compris entre 0 et 3.

**10.** Procédé selon la revendication 8 ou 9, dans lequel X représente

- un groupe aliphatique, linéaire ou ramifié, ou un groupe aralkyle, un groupe alkenyle, cycloalkenyle, cycloalkyle ou cycloalkenyle, tous ces groupes comprenant avantageusement entre 1 à 36 atomes de carbone, et préférentiellement un groupe méthyle, éthyle, propyle, isopropyle, t-butyle, tridécyle, cyclohexyle, 4-pentadécyloxybenzyle, hexadécyloxycarbonyléthyle, 2-éthoxytridécyle, trifluoro-méthyle ou cyclopentyle ; ou
- un groupe alkoxy (de préférence avec 1 à 30 atomes de carbone, tel que méthoxy, éthoxy, 2-méthylméthoxy et 2-dodécyloxyéthoxy), un groupe alkyle, un groupe carbamoyloxy (de préférence avec 1 à 30 atomes de carbone, tel que N-ethyl-carbamoyloxy et N-phénylcarbamoyloxy), un groupe silyloxy (de préférence avec 1 à 30 atomes de carbone, tel que triméthylsilyloxy et dibutylméthylsilyloxy), un groupe acylamino (de préférence avec 2 à 30 atomes de carbone tel que acétamide, tetradécanamide, 2-(2,4-di-t-amylphénoxy)-acétamide, isopentadécanamide), un groupe alkylamino (de préférence avec 1 à 30 atomes de carbone, tel que méthylamino, butylamino, dodécylamino, diméthylamino, diéthylamino et méthylbutylamino), un groupe uréide (de préférence avec 2 à 30 atomes de carbone, tel que méthyluréide, phényluréide, N,N-dibutyluréide et diméthyluréide), un groupe alkényloxy (de préférence avec 2 à 30 atomes de carbone, tel que 2-propenyloxy), un groupe formyle, alkylacyle ou alkyl oxycarbonyl, un groupe alkyl oxycarbonylamino ou un groupe carbamoyle (de préférence avec 1 à 30 atomes de carbone tel que N-éthylcarbamoyle, N,N-dibutylcarbamoyle, N-(2-dodécyloxyéthyl)carbamoyle, N-méthyl-N-dodécylcarbamoyl et N-[3-(2,4-di-t-amylphénoxy)propyl] carbamoyle), un groupe phosphonyle (de préférence avec 1 à 30 atomes de carbone tel que phénoxyphosphonyle, octyloxyphosphonyle et phényl-phosphonyle), un groupe imide (de préférence avec 1 à 30 atomes de carbone tel que N-succinimide, hydantoinyl, N-phthalimide et 3-octadécenylsuccinimide), un groupe azolyle (tel qu'imidazolyle, pyrazolyle, 3-chloro-pyrazol-1-yl et triazolyle), un atome halogène (tel que chlore et brome), un groupe hydroxyle, cyanure, carboxyle ou nitro, ou un groupe amine linéaire ;

ou de manière préférée,

- un groupe alkyle linéaire ou ramifié avec 1 à 15 atomes de carbone et encore plus préférentiellement avec 1 à 8 atomes de carbone, un groupe aralkyle avec 7 à 15 atomes de carbone et encore plus préférentiellement avec 7 ou 8 atomes de carbone, ou un groupe cycloalkyle avec 3 à 15 atomes de carbone et encore plus préférentiellement avec 5 à 8 atomes de carbone ;

ou de manière particulièrement préférée,

- un groupe méthyle, éthyle, n-propyle, i-propyle, n-butyle, s-butyle, t-amyle, t-octyle, phényle ou cyclohexyle.

## Claims

**1.** Continuous method for the catalytic hydrogenation of an aromatic compound, preferably a mono- or polysubstituted benzene derivative, into a cyclo-aliphatic compound, said method being performed in a piston reactor, preferably having a cylindrical shape, said reactor being provided with mechanical axial stirring means, and wherein

- at least one liquid phase comprising said aromatic compound and a catalyst dispersed in liquid phase are continuously introduced, preferably at one end of said reactor,
- said liquid phase is subjected, at a temperature between 100°C and 300°C and under axial mechanical stirring, to the effect of a hydrogen pressure between 10 and 250 bar, and preferentially between 50 and 250 bar, in the presence of said catalyst dispersed in liquid phase, for a passage time t between 1 second and 10 minutes, preferentially between 10 seconds and 6 minutes, and more preferentially between 40 seconds and 3 minutes,
- the liquid phase is taken out of said reactor,

and wherein, preferably, the temperature rise $\Delta T$ of the liquid between the reactor inlet and outlet is such that the ratio $\Delta T$ / $\Delta T_{ad}$ (where $\Delta T_{ad}$ represents the adiabatic temperature rise) is between 0.02 and 0.6 when the ratio between the characteristic heat transfer time $t_{therm}$ and the characteristic material transfer time $t_{mat}$ is between 1 and 50.

**2.** Method according to claim 1, **characterised in that** $\Delta T$ / $\Delta T_{ad}$ is between 0.02 and 0.2 when $t_{therm}$ / $t_{mat}$ is between 1.5 and 12.

**3.** Method according to claim 1, **characterised in that** $\Delta T$ / $\Delta T_{ad}$ is between 0.03 and 0.15 when $t_{therm}$ / $t_{mat}$ is between 2 and 8.

**4.** Method according to any of claims 1 to 3, wherein 3 s < $t_{mat}$ < 10 s.

**5.** Method according to any of claims 1 to 4, wherein the heat transfer is between 300 and 700 W/m$^2$/°C.

**6.** Method according to any of claims 1 to 5, wherein said aromatic compound is the liquid phase introduced into the reactor.

**7.** Method according to any of claims 1 to 5, wherein said aromatic compound is a mono- or polysubstituted phenol, or a non-substituted polyhydroxybenzene or a mono- or polysubstituted polyhydroxybenzene.

**8.** Method according to claim 7, wherein said aromatic compound is as represented by formula (I), and said cyclo-aliphatic compound is as represented by formula (II),

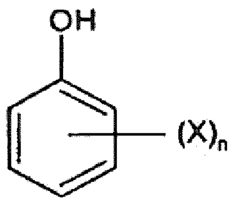 (I)

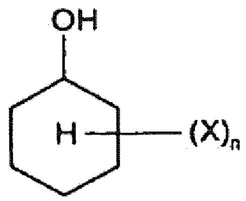 (II)

where X is a substituent which is not a hydrogen atom, and n is an integer between 0 and 3.

**9.** Method according to claim 7, wherein said aromatic compound is as represented by formula (III), and said cyclo-aliphatic compound is as represented by formula (IV),

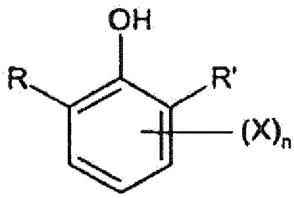 (III) 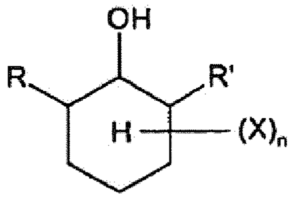 (IV)

where R and R' are, independently from each other, a tertiary alkyl group, X is a substituent which is not a hydrogen atom, and n is an integer between 0 and 3.

**10.** Method according to claim 8 or 9, wherein X represents:

- a linear or branched aliphatic group, or an aralkyl group, an alkenyl, cycloalkenyl, cycloalkyl or cycloalkenyl group, all these groups advantageously comprising between 1 and 36 carbon atoms, and preferentially a methyl, ethyl, propyl, isopropyl, t-butyl, tridecyl, cyclohexyl, 4-pentadecyloxybenzyl, hexadecyloxycarbonyl-ethyl, 2-ethoxytridecyl, trifluoromethyl or cyclopentyl group; or
- an alkoxy group (preferably with 1 to 30 carbon atoms, such as methoxy, ethoxy, 2-methylmethoxy and 2-dodecyloxyethoxy), an alkyl group, a carbamoyloxy group (preferably with 1 to 30 carbon atoms, such as N-ethyl-carbamoyloxy and N-phenylcarbamoyloxy), a silyloxy group (preferably with 1 to 30 carbon atoms, such as trimethylsilyloxy and dibutylmethylsilyloxy), an acylamino group (preferably with 2 to 30 carbon atoms such as acetamide, tetradecanamide, 2-(2,4-di-t-amylphenoxy)-acetamide, isopentadecanamide), an alkylamino group (preferably with 1 to 30 carbon atoms, such as methylamino, butylamino, dodecylamino, dimethylamino, diethylamino and methylbutylamino), a ureide group (preferably with 2 to 30 carbon atoms, such as methylureide, phenylureide, N,N-dibutylureide and dimethylureide), an alkenyloxy group (preferably with 2 to 30 carbon atoms, such as 2-propenyloxy), a formyl, alkylacyl or alkyl oxycarbonyl group, an alkyl oxycarbonylamino or a carbamoyl group (preferably with 1 to 30 carbon atoms such as N-ethylcarbamoyl, N,N-dibutylcarbamoyl, N-(2-dodecyloxyethyl)carbamoyl, N-methyl-N-dodecylcarbamoyl, N-(2-dodecyloxyethyl)carbamoyl, N-methyl-N-dodecylcarbamoyl and N-[3-(2,4-di-t-amylphenoxy)propyl] carbamoyl), a phosphonyl group (preferably with 1 to 30 carbon atoms such as phenoxyphosphonyl, octyloxyphosphonyl and phenylphosphonyl), an imide group (preferably with 1 to 30 carbon atoms such as N-succinimide, hydantoinyl, N-phthalimide and 3-octadecenylsuccinimide), an azolyl group (such as imidazolyl, pyrazolyl, 3-chloro-pyrazol-1-yl and triazolyl), a halogen atom (such as chlorine and bromine), a hydroxyl, cyanide, carboxyl or nitro group, or a linear amine group;

or preferably,

- a linear or branched alkyl group with 1 to 15 carbon atoms and more preferentially with 1 to 8 carbon atoms, an aralkyl group with 7 to 15 carbon atoms and more preferentially with 7 or 8 carbon atoms, or a cycloalkyl group with 3 to 15 carbon atoms and more preferentially with 5 to 8 carbon atoms;

or particularly preferably,

- a methyl, ethyl, n-propyl, i-propyl, n-butyl, s-butyl, t-amyl, t-octyl, phenyl or cyclohexyl group.

## Patentansprüche

**1.** Kontinuierliches Verfahren zur katalytischen Hydrierung einer aromatischen Verbindung, vorzugsweise eines mono- oder polysubstituierten Benzolderivats, zu einer cycloaliphatischen Verbindung, welches Verfahren in einem Kolbenreaktor durchgeführt wird, der vorzugsweise zylindrisch ausgebildet ist, wobei der Reaktor mit einem axial angeordneten mechanischen Rührmittel ausgestattet ist,
und wobei

- ihm kontinuierlich, vorzugsweise an einem Ende des Reaktors, mindestens eine flüssige Phase zugeführt wird, welche die aromatische Verbindung und einen in flüssiger Phase dispergierten Katalysator enthält,
- die flüssige Phase bei einer Temperatur zwischen 100 °C und 300 °C und unter mechanischem Rühren in axialer Richtung mit einem Wasserstoffdruck zwischen 10 und 250 bar, vorzugsweise zwischen 50 und 250 bar beaufschlagt wird, und zwar in Gegenwart des in flüssiger Phase dispergierten Katalysators für eine Übergangszeit t zwischen 1 Sekunde und 10 Minuten, vorzugsweise zwischen 10 Sekunden und 6 Minuten und besonders bevorzugt zwischen 40 Sekunden und 3 Minuten,
- die flüssige Phase aus dem Reaktor herausgeführt wird,

und wobei die Temperaturerhöhung $\Delta T$ der Flüssigkeit zwischen Eingang und Ausgang des Reaktors vorzugsweise so gewählt wird, dass das Verhältnis $\Delta T$ / $\Delta T_{ad}$ (wobei $\Delta T_{ad}$ die adiabatische Temperaturerhöhung darstellt) 0,02 bis 0,6 beträgt, wenn das Verhältnis zwischen charakteristischer Wärmeübertragungszeit $t_{therm}$ und charakteristischer Stoffaustauschzeit $t_{mat}$ im Bereich zwischen 1 und 50 liegt.

**2.** Verfahren nach Anspruch 1, **dadurch gekennzeichnet, dass** $\Delta T$ / $\Delta T_{ad}$ 0,02 bis 0,2 beträgt, wenn $t_{therm}$ / $t_{mat}$ im Bereich zwischen 1,5 und 12 liegt.

**3.** Verfahren nach Anspruch 1, **dadurch gekennzeichnet, dass** $\Delta T$ / $\Delta T_{ad}$ 0,03 bis 0,15 beträgt, wenn $t_{therm}$ / $t_{mat}$ im Bereich zwischen 2 und 8 liegt.

**4.** Verfahren nach einem der Ansprüche 1 bis 3, wobei 3 s < $t_{mat}$ < 10 s.

**5.** Verfahren nach einem der Ansprüche 1 bis 4, wobei die Wärmeübertragung 300 bis 700 $W/m^2/°C$ beträgt.

**6.** Verfahren nach einem der Ansprüche 1 bis 5, wobei die aromatische Verbindung die in den Reaktor eintretende flüssige Phase darstellt.

**7.** Verfahren nach einem der Ansprüche 1 bis 5, wobei die aromatische Verbindung ein mono- oder polysubstituiertes Phenol oder ein unsubstituiertes oder mono- oder polysubstituiertes Polyhydroxybenzol ist.

**8.** Verfahren nach Anspruch 7, wobei die aromatische Verbindung den durch die Formel (I) dargestellten Aufbau hat, und die cycloaliphatische Verbindung den durch die Formel (II) dargestellten Aufbau hat,

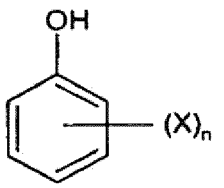
(I)

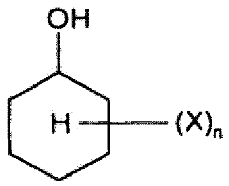
(II)

worin X ein Substituent ist, der kein Wasserstoffatom ist, und n eine ganze Zahl zwischen 0 und 3 ist.

**9.** Verfahren nach Anspruch 7, wobei die aromatische Verbindung den durch die Formel (III) dargestellten Aufbau hat, und die cycloaliphatische Verbindung den durch die Formel (IV) dargestellten Aufbau hat,

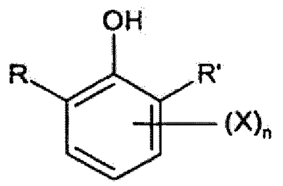
(III)

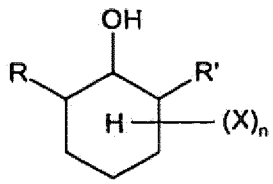
(IV)

worin R und R' unabhängig voneinander eine tertiäre Alkylgruppe sind, X ein Substituent ist, der kein Wasserstoffatom ist, und n eine ganze Zahl zwischen 0 und 3 ist.

**10.** Verfahren nach Anspruch 8 oder 9, wobei X darstellt:

- eine lineare oder verzweigte aliphatische Gruppe, eine Aralkylgruppe oder eine Alkenyl-, Cycloalkenyl-, Cycloalkyl- oder Cycloalkenylgruppe, wobei alle diese Gruppen mit Vorteil 1 bis 36 Kohlenstoffatome aufweisen, und vorzugsweise eine Methyl-, Ethyl-, Propyl-, Isopropyl-, t-Butyl-, Tridecyl-, Cyclohexyl-, 4-Pentadecyloxybenzyl-, Hexadecyloxycarbonylethyl-, 2-Ethoxytridecyl-, Trifluormethyl- oder Cyclopentylgruppe; oder
- eine Alkoxygruppe (vorzugsweise mit 1 bis 30 Kohlenstoffatomen, wie Methoxy, Ethoxy, 2-Methylmethoxy und 2-Dodecyloxyethoxy), eine Alkylgruppe, eine Carbamoyloxygruppe (vorzugsweise mit 1 bis 30 Kohlenstoffatomen, wie N-Ethylcarbamoyloxy und N-Phenylcarbamoyloxy), eine Silyloxygruppe (vorzugsweise mit 1

bis 30 Kohlenstoffatomen, wie Trimethylsilyloxy und Dibutylmethylsilyloxy), eine Acylaminogruppe (vorzugsweise mit 2 bis 30 Kohlenstoffatomen, wie Acetamid, Tetradecanamid, 2-(2,4-Di-t-amylphenoxy)-acetamid, Isopentadecanamid), eine Alkylaminogruppe (vorzugsweise mit 1 bis 30 Kohlenstoffatomen, wie Methylamino, Butylamino, Dodecylamino, Dimethylamino, Diethylamino und Methylbutyoamino), eine Ureidgruppe (vorzugsweise mit 2 bis 30 Kohlenstoffatomen, wie Methylureid, Phenylureid, N,N-Dibutylureid und Dimethylureid), eine Alkenyloxygruppe (vorzugsweise mit 2 bis 30 Kohlenstoffatomen, wie 2-Propenyloxy), eine Formyl-, Alkylacyl- oder Alkyloxycarbonylgruppe, eine Alkyloxycarbonylaminogruppe oder eine Carbamoylgruppe (vorzugsweise mit 1 bis 30 Kohlenstoffatomen, wie N-Ethylcarbamoyl, N,N-Dibutylcarbamoyl, N-(2-Dodecyloxyethyl)-carbamoyl, N-Methyl-N-dodecylcarbamoyl und N-[3-(2,4-Di-t-amylphenoxy)-propyl]carbamoyl), eine Phosphonylgruppe (vorzugsweise mit 1 bis 30 Kohlenstoffatomen, wie Phenoxyphosphonyl, Octyloxyphosphonyl und Phenylphosphonyl), eine Imidgruppe (vorzugsweise mit 1 bis 30 Kohlenstoffatomen, wie N-Succinimid, Hydantoinyl, N-Phthalimid und 3-Octadecenylsuccinimid), eine Azolylgruppe (wie Imidazolyl, Pyrazolyl, 3-Chloropyrazol-1-yl und Triazolyl), ein Halogenatom (wie Chlor und Brom), eine Hydroxyl-, Cyanid-, Carboxyl- oder Nitrogruppe, oder eine lineare Amingruppe;
oder bevorzugt
- eine lineare oder verzweigte Alkylgruppe mit 1 bis 15 Kohlenstoffatomen und besser noch mit 1 bis 8 Kohlenstoffatomen, eine Aralkylgruppe mit 7 bis 15 Kohlenstoffatomen und besser noch mit 7 oder 8 Kohlenstoffatomen, oder eine Cycloalkylgruppe mit 3 bis 15 Kohlenstoffatomen und besser noch mit 5 bis 8 Kohlenstoffatomen;
oder insbesondere bevorzugt
- eine Methyl-, Ethyl-, n-Propyl-, i-Propyl-, n-Butyl-, s-Butyl- t-Amyl- t-Octyl-, Phenyl oder Cyclohexylgruppe.

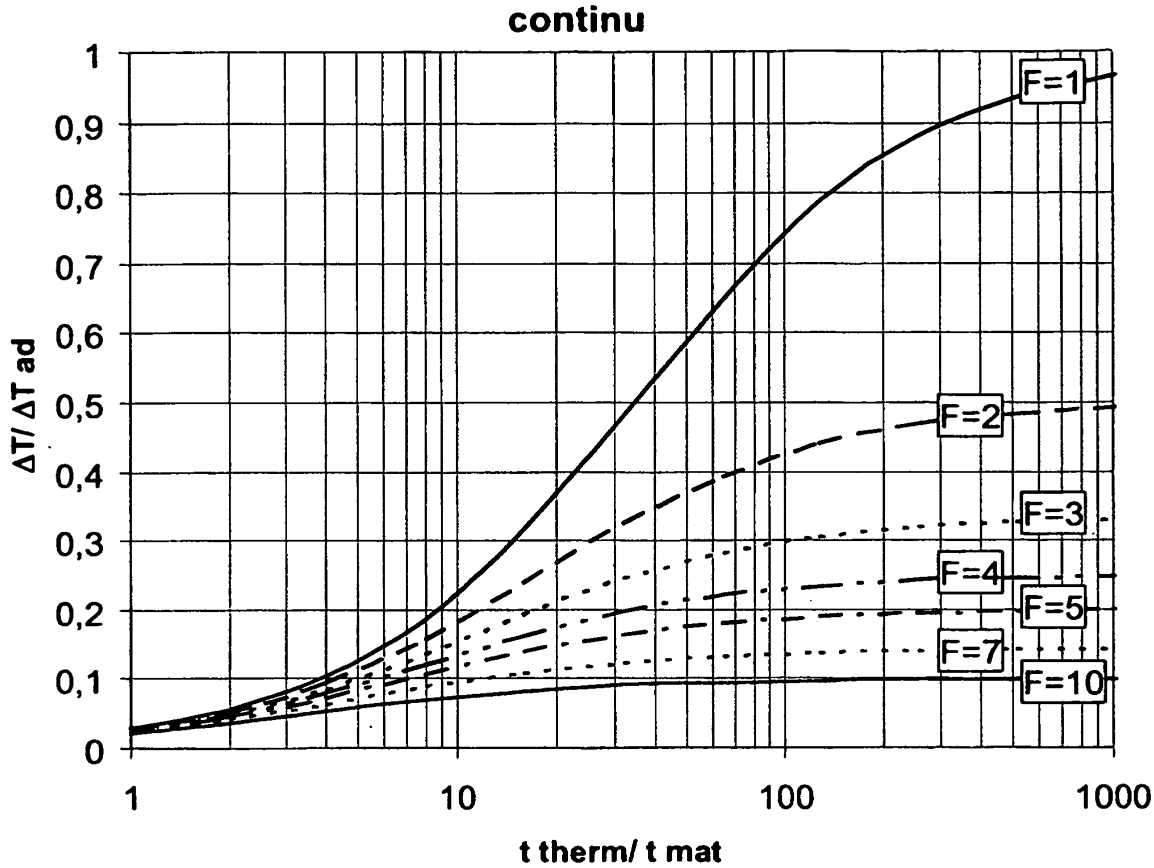
continu
1
0,9
0,8
0,7
0,6
0,5
0,4
0,3
0,2
0,1
0
ΔT/ ΔT ad
1
10
100
1000
t therm/ t mat
F=1
F=2
F=3
F=4
F=5
F=7
F=10

Figure 1

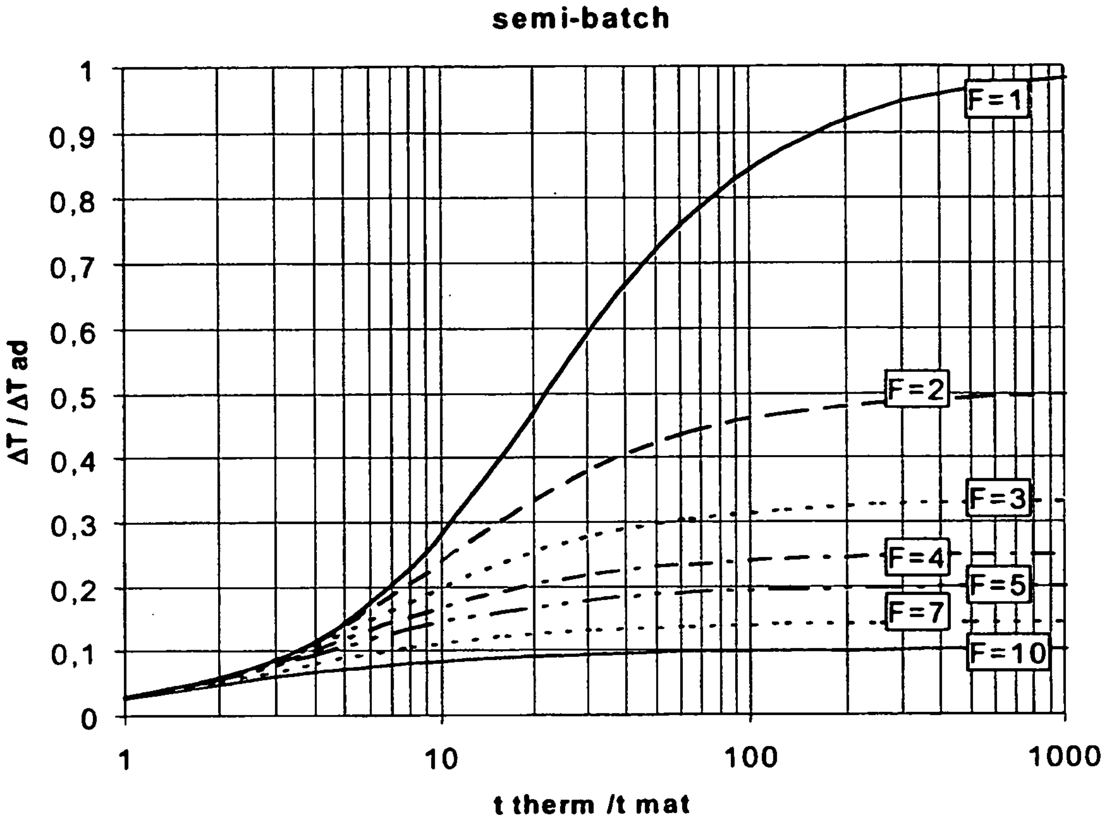
semi-batch
1
0,9
0,8
0,7
0,6
0,5
0,4
0,3
0,2
0,1
0
ΔT / ΔT ad
1
10
100
1000
t therm /t mat
F=1
F=2
F=3
F=4
F=5
F=7
F=10

Figure 2

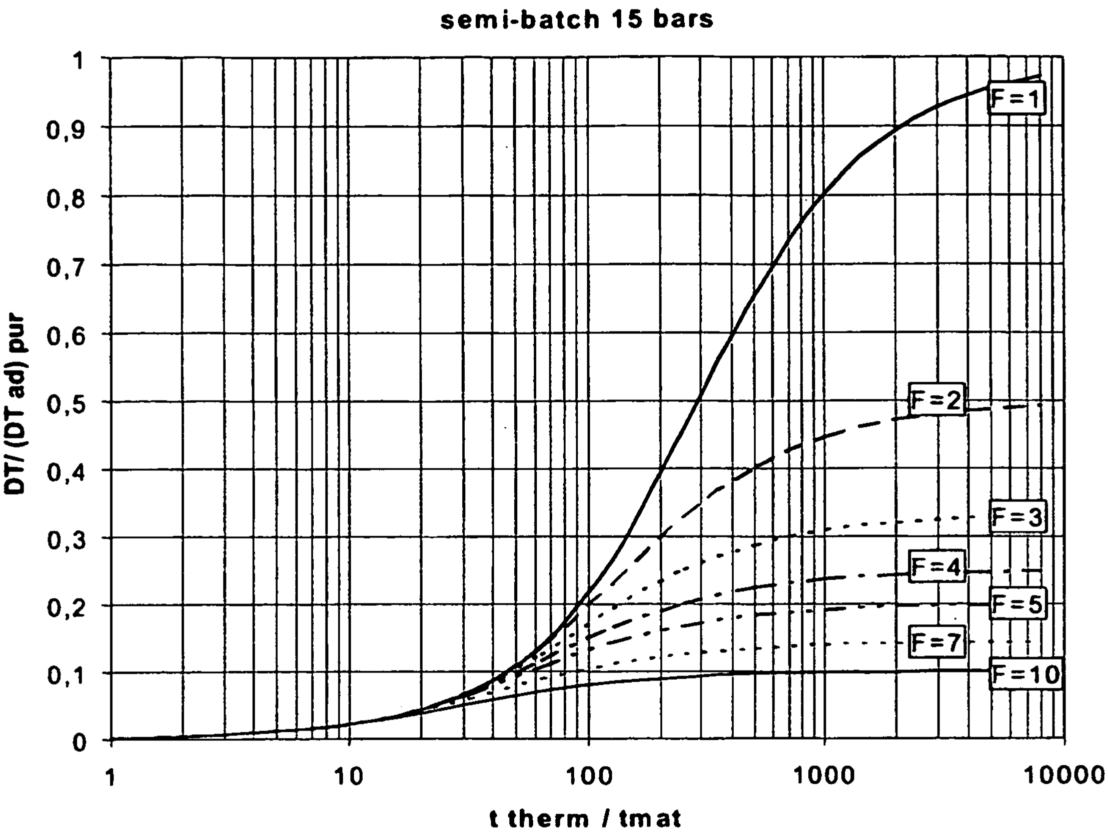
semi-batch 15 bars
1
0,9
0,8
0,7
0,6
0,5
0,4
0,3
0,2
0,1
0
DT/ (DT ad) pur
1
10
100
1000
10000
t therm / tmat
F=1
F=2
F=3
F=4
F=5
F=7
F=10

Figure 3

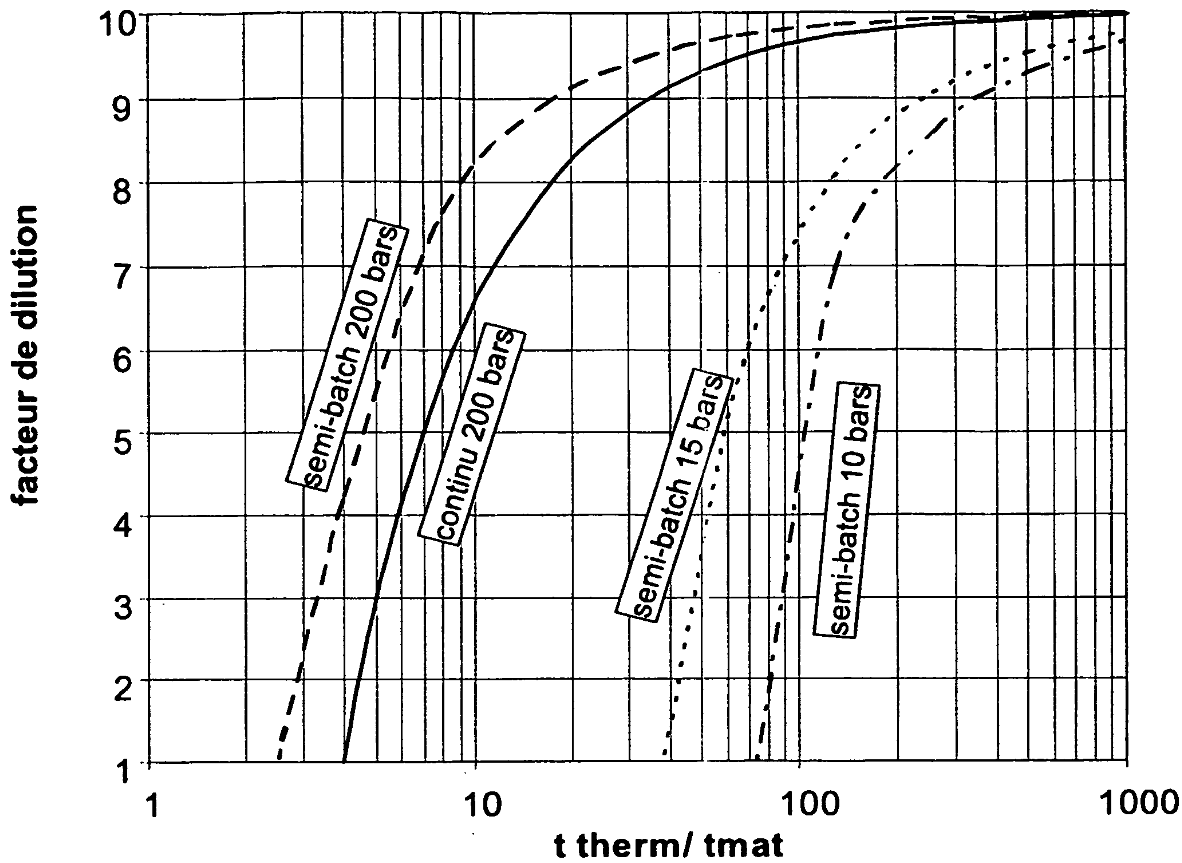
10
9
8
7
6
5
4
3
2
1
facteur de dilution
1
10
100
1000
t therm/ tmat
semi-batch 200 bars
continu 200 bars
semi-batch 15 bars
semi-batch 10 bars

Figure 4

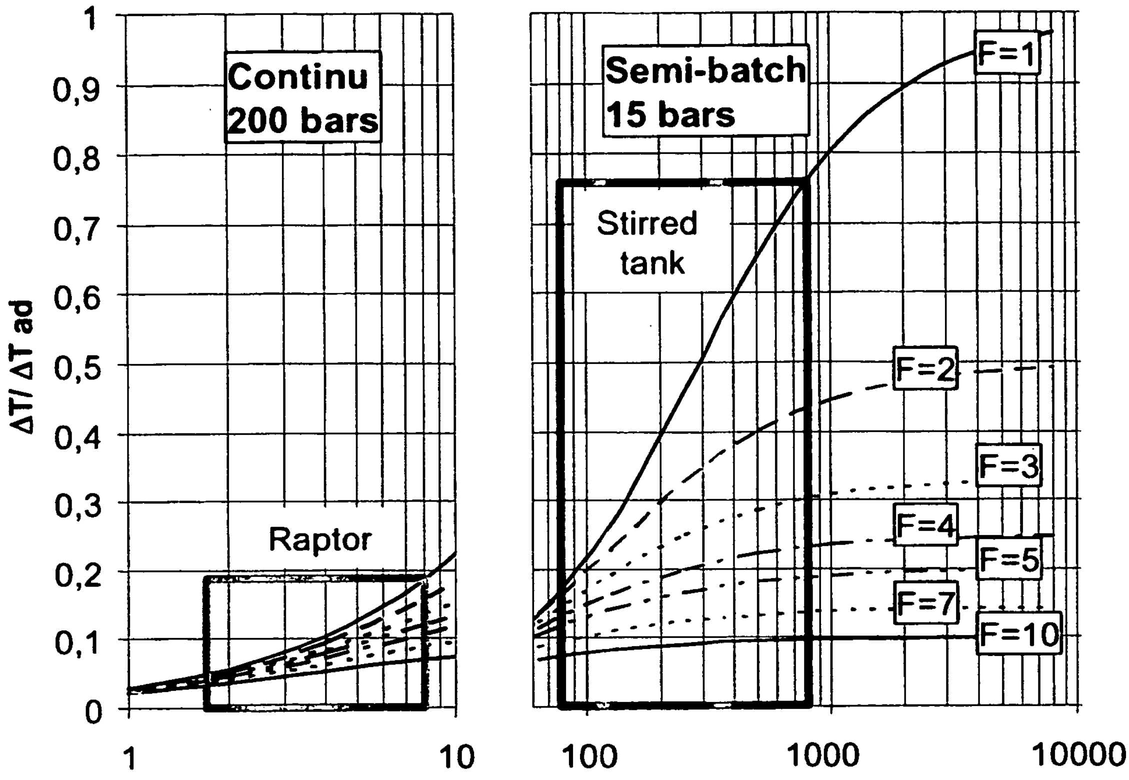

1
0,9
0,8
0,7
0,6
0,5
0,4
0,3
0,2
0,1
0
ΔT/ ΔT ad
Continu
200 bars
Raptor
Semi-batch
15 bars
Stirred
tank
F=1
F=2
F=3
F=4
F=5
F=7
F=10
1
10
100
1000
10000


Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2398709
- US 2857432 A
- FR 2217293
- US 6489520 B
- EP 0703210 A1
- EP 0427965 A
- US 5874648 A
- US 6031140 A
- US 5942645 A
- US 5189233 A
- US 4551564 A
- FR 2040257
- US 3700742 A